# EUROPEAN PATENT SPECIFICATION

(11) **EP 4 582 014 B1**
(45) Date of publication and mention of the grant of the patent: **11.11.2026**
(21) Application number: 24217602.2
(22) Date of filing: 04.12.2024
(51) Int. Cl.: A61B 5/00, A61M 21/02, A61M 21/00

(54) **SYSTEM AND METHODS FOR IMPROVING SLEEP QUALITY**
SYSTEM UND VERFAHREN ZUR VERBESSERUNG DER SCHLAFQUALITÄT
SYSTÈME ET PROCÉDÉS POUR AMÉLIORER LA QUALITÉ DU SOMMEIL

(30) Priority: 03.01.2024 GB 202400080
(43) Date of publication of application: 09.07.2025
(73) Proprietor: Inoveris Solutions SRL, 400495 Cluj-Napoca (RO)
(72) Inventor: FRATILA, Adrian, 400495 Cluj-Napoca (RO)
(74) Representative: Maidment, Marc

(56) References cited:
- US-A1- 2019 223 612
- US-A1- 2019 290 011
- US-A1- 2022 265 207
- US-A1- 2023 302 253
- US-B2- 7 007 327

## Description

The present invention generally relates to a method of controlling a sleep enhancing device or system, for improving sleep quality for a sleeping person; a system for carrying out that method; and a method of generating, by a machine learning model, control data for controlling a sleep enhancing device or system for improving sleep quality.

### BACKGROUND TO THE INVENTION

Sleep is essential for everyone, but it does not always come easily. A person may have difficulty getting to sleep, difficulty staying asleep, difficulty waking up, difficulty getting back to sleep after waking up, or may still feel tired after waking up, or a combination of any of these.

Sleep interruptions may occur sporadically due to an illness or as a result of snoring, for example, but disrupted sleep resulting from a sleep disorder can be extremely debilitating and can affect all areas of a person's life. There are over 80 different sleep disorders known at present.

Other people may have no trouble sleeping *per se,* but may have busy personal and/or working lives that place many demands on their time. Most people require 7-8 hours of sleep per day but may not regularly achieve that amount of sleep, leaving them with sleep debt that can accumulate and may be debilitating in its own way.

Somnology is the scientific study of sleep and may be used to help people who experience disrupted sleep, particularly in relation to sleep disorders. Somnology divides sleep into four distinct stages/periods, referred to as sleep stage 1 (N1), sleep stage 2 (N2), sleep stage 3 (N3) and sleep stage 4 (REM), which are discussed further below.

During sleep, there is a progressive passage through the stages of sleep from stage 1 (N1) to sleep stage 4 (REM), which forms a sleep cycle, after which the cycle resumes from sleep stages 2 to 4. A complete sleep cycle in adults lasts between 90 to 110 minutes. The first or initial sleep cycles include relatively short periods of REM sleep and relatively long periods of deep sleep. During the night, the periods of REM sleep increase in length, and those of deep sleep become shorter. A person who is asleep for a night will generally have four to six sleep cycles, meaning that they experience the various sleep stages several times.

Considering the sleep stages in more detail, the first sleep stage is stage 1, which is considered to be 'light' sleep where the body has not fully relaxed yet. Sleep stage 1 (N1) can last about 5 to 10 minutes and represents the beginning of the transition from wakefulness to sleep.

The next sleep stage 2 (N2) can last for 10 to 25 minutes during the first sleep cycle (i.e. the first instance of sleep stage 2 after having fallen asleep). Each sleep stage 2 can become longer during the course of the person's time asleep. In total, a person typically spends about 50% of their total sleep in sleep stage 2. Eye movements stop and brain waves slow down more during sleep stage 2.

Sleep stage 3 (N3) is deep sleep and represents approximately 20% of the total sleep time. Sleep stage 3 is the most important for rest and regeneration. In this phase, no muscle activity is registered, and the brain emits slow brain waves, called delta waves, associated with states of rest and deep regeneration. A healthy person spends the longest time in sleep stage 3 during the first half of the night. During the initial sleep cycles, sleep stage 3 usually lasts for 20 to 40 minutes. Throughout the night, instances of sleep stage 3 become shorter and more time is spent in stage 4 instead.

Sleep stage 4 is REM sleep (where REM means rapid eye movement) and represents approximately 25% of the total sleep time. Sleep stage 4 is the stage in which dreams occur. Sleep stage 4 plays a role in strengthening memory, processing emotions, and general regeneration of the body.

During sleep stage 4 (REM), the brain functions in many ways that are similar to when awake. The brain waves are active, and the breathing and heart rate increase and become more variable than in other sleep stages, which implies that the body does not rest during the REM sleep. If the amount of time spent in REM sleep during the night is too long, which implies a lack of deep sleep in sleep stage 3, the subject will feel tired in the morning.

Although REM sleep was previously believed to be the most important sleep phase for learning and memory, newer studies have suggested that non-REM sleep (that is, stages 1, 2 and 3) are more important in this respect, leaving aside the advantage that these stages are also more restful and restorative than REM sleep. Given that there is a surge in activity during REM sleep, this may explain why already vulnerable people often experience heart attacks and other events in the early morning hours, which is typically spent more in REM sleep.

There are scientific studies that have shown applying a slow oscillating movement to a bed reduces the time taken to initiate sleep, as well as increasing the time spent in a deeper state of sleep, and can lead to improvements in a person's memory capabilities. The Applicant's patent application WO2018083153 discloses a method for controlling a rocking bed where the bed rocking motion is stopped if the user leaves the bed or if the user falls asleep (e.g. because they have stopped moving).

However, each person has a different sensitivity to rocking or oscillatory motion. If this movement is in excess for a given person, there is a risk that that person will feel dizzy or even sick or nauseous. This is far from ideal for a relatively fit and healthy person, and may present a real danger to an elderly or more vulnerable person.

US2019/0223612 A1 relates to a device for providing a kinetic input to a user. The device includes a rockable bed frame which includes a top portion, a bottom portion, and a linear drive unit positioned between the top portion and the base portion to move the top portion and base portion relative to one another.

US2023/0302253 A1 relates to systems, methods, and devices for measurement, identification, and generation of sleep state models. Systems include a plurality of electrodes configured to be coupled to a brain of a user and configured to obtain a plurality of measurements from the brain of the user, and an interface configured to obtain the plurality of measurements from the plurality of electrodes. Systems include a processing device comprising one or more processors configured to generate a sleep state model associated with the user, the sleep state model identifying characteristics of a plurality of sleep stages, and further identifying characteristics of transitions between the plurality of sleep stages. Systems include a controller comprising one or more processors configured to generate a control signal based on the sleep state model and the plurality of measurements.

US7007327 B2 relates to an adjustable bed that assists a user to turn comfortably in his or her sleep without mental stress. Once an automatic mode starts, measurement on the depth of sleep of the user lying on the bed is launched. The depth of sleep is measured by detecting biological information such as brain waves, heart rate, and respiratory rate of the user using electrode sensors or the like, and analysing the biological information according to a polysomnography method or the like. If the depth of sleep is 0, i.e., the user is in REM sleep, and if one hour or more has passed since the measurement is launched or since an immediately preceding turn operation, a turn operation is performed to turn the user.

It is an object of the present invention to reduce or substantially obviate the aforementioned problems.

### STATEMENT OF INVENTION

The object of the present invention is achieved with a system or a non-transitory computer readable medium according to the independent claims.

According to a first aspect of the present disclosure, there is provided a method of controlling a sleep enhancing device or system (e.g. on or next to a bed), for improving sleep quality for a person, the method comprising the steps of:
estimating, based on information from at least one or more sensors, which sleep stage the person is in, the stage of sleep being selected from sleep stage 3 (N3), sleep stage 4 (REM) and another sleep stage (optionally including sleep stage 1 (N1) and/or sleep stage 2 (N2)); and
when the person is estimated to be in sleep stage 3 (N3) and/or when the person is estimated to be in sleep stage 4 (REM) (and optionally when they are estimated to be in sleep stage 1 (N1) and/or sleep stage 2 (N2)), performing one of:
   starting the sleep enhancing device or system to begin providing an input which acts on the person during the estimated sleep stage(s);
   stopping the sleep enhancing device or system to cease providing an input which is acting on the person during the estimated sleep stage(s);
   adjusting (or optionally maintaining) the sleep enhancing device or system to change the level of an input which is acting on the person during the estimated sleep stage(s).

This has major advantages for improving overall sleep quality, such as allowing a person to have more restorative sleep. This can prolong sleep duration in a particular sleep state and/or reduce sleep duration in another particular sleep state. That is, the time spent in one sleep stage can be controlled relative to the time spent in another sleep stage.

Put another way, it allows for the time spent in deep sleep and/or REM to be maximised. In particular, it can allow a person to have a controllably reduced proportion of their time spent asleep whilst in sleep stage 1 (N1) and sleep stage 2 (N2) relative to sleep stage 3 (N3) and/or sleep stage 4 (REM). For example, it may allow a person to achieve or reach sleep stage 3 (N3) more quickly than would otherwise occur.

The method aims to maintain a state of deep sleep for the bed user, while minimising possible adverse effects experienced due to the bed oscillatory motion. That is, the bed motion is tailored to the person and/or controlled (preferably automatically) in a way that depends on the sleep stage that the person is estimated to be in.

The instruction or decision to start and/or stop and/or adjust the input provided by the device/system is preferably determined by an artificial intelligence (AI) algorithm or machine learning model that has been trained on a suitable number of data sets. In general, the larger the number of data sets used for training, the better the training outcome for the resulting model. The number of data sets may be about at least about 10000, for example.

Based on information provided by sensors which are sensing or periodically/continuously monitoring vital parameters of the person's body, the algorithm or model can precisely estimate of the sleep stage that the person is in and provide an instruction / decision accordingly.

The invention may utilise Al algorithm(s) which, based on information received from sensors during a person's time asleep, can learn the patterns and/or sleep stages of a person's sleep cycles (for example based on data acquired over several nights of that person's sleep). Based on that information, the Al algorithm(s) may then make predictions and determinations as to when an input from a sleep enhancing device/system ought to be started and/or stopped and/or adjusted for improving that person's sleep quality.

The invention is suitable for any person who suffers from a sleep disorder and/or from any secondary health effects that have arisen or worsened due to a lack of sleep.

The invention is also suitable for any person who does not have any sleep problems/disorders, for example if they wish to reduce their total required sleep time. That is, to achieve a restful sleep in a shorter time.

In preferred embodiments, the time spent by a sleeping person in sleep stages 1 and 2 should be reduced to a minimum, whilst the time spent in sleep stage 3 should be quickly entered, maintained for longer than normal and alternated with sleep stage 4 (REM) which is important for the memory regeneration.

The method may therefore prioritise initiation and/or maintenance of sleep stage 3 and provide input(s) to achieve this. The method may also or instead prioritise artificially lengthened and/or artificially ended periods for either or both of sleep stages 3 and 4 for controlled transfer of the person between sleep stages 3 and 4, whilst minimising time spent in sleep stages 1 and 2, by providing input(s) to achieve this.

Estimates of which particular sleep stage exists at the time of a given sensor reading may be based on confidence scores/values. That is, the sensor reading may be processed or assessed to determine the likelihood that it corresponds to sleep stage 3 (N3) or sleep stage 4 (REM) or another sleep stage or stages, and the sleep stage which has the corresponding highest confidence score/value may be determined as the estimated sleep stage.

Estimates may be made periodically or substantially continuously. For example, the frequency at which estimates are made may depend on the frequency of data sampling by the sensors and/or a user-set value for how frequently the sleep stage estimation should be refreshed or re-estimated.

The sleep enhancing device or system may be a bed rocking device or system. For example, the bed rocking may be achieved using a device/system similar to that presented in the Applicant's patent application WO2018083153 or the Applicant's patent application EP4137008.

The input is a bed rocking motion or a reciprocating motion or an oscillatory motion. Any input value discussed below may correspond to a bed rocking motion speed or a reciprocating motion speed or an oscillatory motion speed. The input value in such cases could vary between 0 (i.e. stopped or at rest) to a maximum value of 100 (which may correspond to a speed of approximately 10mm per second).

If the person is estimated to be in either or both of sleep stage 1 (N1) and sleep stage 2 (N2), the method may include operating (or continuing to operate) the sleep enhancing device(s) or system(s). That is, rather than starting/stopping/adjusting the input, the method may allow the sleep enhancing device/system to continue at its previous input value or in its current state.

The input during sleep stages 1 and/or 2 may be a non-zero input. For example, a bed rocking motion or bed speed may have a non-zero value during any estimated sleep stage 1 and/or sleep stage 2 during the method.

In other words, the bed may move or rock during sleep stage 1 and/or sleep stage 2. The input value in sleep stage 1 and/or sleep stage 2 may be a predetermined or user-set value. That is, for operation of the sleep enhancing device in sleep stages 1 and/or 2 the input may have a value preset according to user choice, and for operation of the sleep enhancing device in sleep stages 3 and/or 4 the input value may be set or varied according to control data which may be or have been generated by a machine learning model.

Rocking the bed up to a certain speed during sleep stages 1 and/or 2 can help the person to reach sleep stage 3 (N3) more quickly, at which point the bed rocking motion may be reduced or ceased.

The method may include estimating, based on information from at least the one or more sensors, when the person enters or commences sleep stage 3 (N3) and/or sleep stage 4 (REM). The method may include stopping or adjusting the input provided by the sleep enhancing device or system when the person is estimated to have entered sleep stage 3 (N3) or sleep stage 4 (REM).

In other words, when the user is in a deep sleep, the movement of the bed is preferably reduced (optionally gradually reduced) to a minimum or stopped. This can reduce possible adverse effects resulting from the sleep enhancing device or system, such as adverse effects from an oscillatory / rocking movement.

The method may include estimating whether sleep stage 3 (N3) and/or sleep stage 4 (REM) has elapsed by a predetermined extent. The method may include starting or adjusting (optionally gradually starting/adjusting) the input provided by the sleep enhancing device or system when the relevant sleep stage is estimated to have elapsed by the relevant predetermined extent.

This can help to prolong sleep stage 3 and/or end sleep stage 4, relative to what would otherwise happen for the person during non-augmented sleep.

The predetermined extent for sleep stage 3 (N3) may correspond to a few minutes (perhaps up to 10 minutes) before a predicted end of that sleep stage 3.

The predicted end may be based on a determined success rate of previous input starts prior to the end of the sleep stage 3. For example, an input (re)start during sleep stage 3 may occur incrementally or progressively earlier, that is prior to the predicted end of sleep stage 3, in subsequent sleep stages 3 if the input start of a previous sleep stage does not have the desired effect, e.g. prolonging that sleep stage 3.

The sleep enhancing device or system may be programmable with a predetermined input value for sleep stage 3 (N3). The input generated or conveyed by the sleep enhancing device/system may be set at the predetermined input value (optionally gradually incremented to that value) at around the predicted end of the sleep stage 3 (N3).

This allows for a user-defined input value such as bed rocking speed to be realised according to the user's sensitivity to the input, that is what they are comfortable with.

Any predetermined input value may be set via a remote control (which may be provided with the sleep enhancing device/system) and/or via an app on an electronic device (e.g. smartphone or tablet) programmed to communicate with the sleep enhancing device/system.

Where the person is estimated to be in sleep stage 3 (N3), the input may be stopped if the duration of that sleep stage 3 (N3) exceeds about 60 minutes or exceeds a predetermined length of time. The predetermined length of time may be a user set value or may be a clinically set value (e.g. determined by a doctor), which may be greater than or less than 60 minutes.

This avoids prolonging sleep stage 3 excessively, in order to achieve an optimum augmented balance with the other sleep stages, and may facilitate alternation with sleep stage 4.

If the input is stopped at the end of sleep stage 3 (N3) and the person next enters sleep stage 4 (REM), then the input may (for a time) be maintained at a zero input value.

If the predetermined input value was insufficient to prolong a first sleep stage 3 (N3) period, then a higher input value may be used during one or more subsequent sleep stage 3 (N3) periods. The input value may be about 5% higher than the predetermined value.

The sleep enhancing device or system may be programmable with a predetermined input start time for sleep stage 3 (N3). The input may start at the predetermined input start time when the person is estimated to be in sleep stage 3 (N3).

These features can variously help to prolong a sleep stage 3 for a desired longer duration in order to provide a beneficially longer period of deep sleep.

The predetermined extent for sleep stage 4 (REM) may correspond to about half of the predicted duration of that sleep stage 4 (REM).

The sleep enhancing device or system may be programmable with a predetermined input value for sleep stage 4 (REM). The input may be set at the predetermined input value at around midway through the sleep stage 4 (REM).

If the predetermined input value was insufficient to end a first sleep stage 4 (REM) period, then a higher input value may be used during one or more subsequent sleep stage 4 (REM) periods. The input value may be about 5% higher than the predetermined value.

The sleep enhancing device or system may be programmable with a predetermined input start time for sleep stage 4 (REM). The input may start at the predetermined input start time when the person is estimated to be in sleep stage 4 (REM).

These features can variously help to controllably end a sleep stage 4 earlier than during non-augmented sleep, and may help facilitate an earlier return to a subsequent sleep stage 3 for the person during a given sleep.

The one or more other sleep stages may include sleep stage 1 (N1) and/or sleep stage 2 (N2). When the person is estimated to be in sleep stage 1 (N1) and/or when the person is estimated to be in sleep stage 2 (N2), the method may include performing at least one of:
starting the sleep enhancing device or system to begin providing an input which acts on the person;
stopping the sleep enhancing device or system to cease providing an input which is acting on the person;
maintaining an input at a level already being provided by the sleep enhancing device or system;
adjusting the sleep enhancing device or system to change the level of an input which is acting on the person.

A person may enter various different sleep stages at various different times during the course of a sleep. It will be appreciated that the method may involve control of the input (whether starting, stopping, maintaining and/or adjusting the input) at various different points of some or all of those sleep stages, which may include control of the input at the commencement of a particular sleep stage (according to an estimate made during the method) and/or control of the input partway through a particular sleep stage (again according to an estimate made during the method) and/or control at the end of a particular sleep stage (again according to an estimate made during the method).

It will also be appreciated that control of the input during a sleep stage may include multiple instances of controlling the input during the course of a single sleep stage.

The sleep enhancing device or system may be programmable with clinical information for the person. Any one or more of the following input parameters may be controlled based at least in part on the clinical information: input type, input intensity, input start time, input end time, input duration, input pattern.

A doctor or other medical / care professional can provide the relevant information to the device/system, which may affect its operation. For example, if the person has a diagnosed sleep condition / disorder then this overrides or takes precedence over what the control data or instructions may contain.

Clinical information may be used by the device/system in a way that seeks to avoid an input exacerbating an existing condition and/or seeks to minimise the likelihood of a condition being triggered. For example, if a user has a cardiovascular-related problem, and might suffer a heart attack during REM sleep, the device/system may reduce the time for which bed rocking is halted during sleep stage 4.

The sleep enhancing device or system may be programmable with a sleep duration value. The sleep duration value may be measured beginning from a clock time or a time when the person is detected as having fallen asleep.

Where a given sleep stage 3 (N3) or sleep stage 4 (REM) is predicted to be the last sleep stage 3 (N3) or last sleep stage 4 (REM) before the sleep duration value is exceeded, the sleep enhancing device or system and the associated input may be stopped and may not restart when the person enters any subsequent sleep stage before they are awake.

The person can thus set a time for which they would like to be asleep and the sleep enhancing device/system operates accordingly. Stopping the input (such as bed rocking) during the last of the predicted deep sleep stages, and not restarting it in subsequent sleep stages prior to waking, can help to avoid making the person feel dizzy when they wake up.

Any suitable sensor may be used. A consumer or medical device (or devices) may integrate sensors for monitoring vital parameters which provide information about the sleep stages. Examples of such devices include any or more of the following for provision in the sleep enhancing system.

A bracelet or wristwatch may be provided which includes sensor(s) for any one, some or all of: motion, temperature, measure heart rate / pulse, electrocardiogram (ECG), blood pressure.

A chest belt may be provided which includes sensor(s) for any one, some or all of: movement, temperature, heart rate/pulse measurement, electrocardiogram (ECG), heart rate variability.

A head band may be provided which includes sensor(s) for any one, some or all of: motion, temperature, and EEG (electro encephalogram).

A mat-type sensor may be provided for placement on or under a mattress. The mat-type sensor may include sensor(s) for any one, some or all of: motion, breathing rate, heart rate / pulse, heart rate variability.

An accelerometer type sensor may be provided for positioning on the mattress for sensing motion. The purpose of this sensor is to sense movement of the person on the mattress, which may happen when the person on the mattress changes their lying position, for example by turning over. The accelerometer type sensor may not be used to sense a lateral movement of the mattress caused by a bed rocking apparatus or device, for example.

All of the above sensors have various advantages and disadvantages due to their positioning in relation to the person's body during sleep. Some of them may be uncomfortable during sleep (such as the chest belt or head band). Whichever device(s) are provided and worn by a person during sleep, the positioning of each device in relation to the person's body and the type of device used affects the accuracy and hence utility of the readings taken of the person's vital parameters.

Since there is presently no universal sensor that suitably senses all of the body's vital parameters, using a combination of multiple sensors (preferably 3-6 or more different sensors) means that the estimates made concerning sleep stage can be substantially more accurate.

The method may be a computer-implemented method.

The method may be performed on a bed or seat where the sleeping person is located.

The input may include a rocking or reciprocating motion.

The method may be performed, at least in part, using control data generated by a machine learning model according to the third aspect of the disclosure for estimating one or more sleep stage parameters.

According to a second aspect of the present invention, there is provided a system for improving sleep quality for a sleeping person, as set out in claim 1. Optional features are set out in the dependent claims.

The advantages are similar to those discussed for the first aspect of the disclosure.

The system may be configured to perform the method of the first aspect of the disclosure. The system may include any feature or features presented with respect to the first aspect of the disclosure.

According to a third aspect of the present disclosure, there is provided a method of generating, by a machine learning model, control data for controlling a sleep enhancing device or system for improving sleep quality, the method comprising the steps of:
providing or accessing a machine learning model trained to estimate a sleep stage at which a sensor reading was acquired including when a sensor reading was acquired during sleep stage (N3) and when a sensor reading was acquired during sleep stage 4 (REM) (and optionally when sensor readings were acquired during either or both of sleep stage 1 (N1) and sleep stage 2 (N2)), or training such a machine learning model;
providing, to the trained machine learning model, a data set of a plurality of sensor readings of a person which have been acquired, or are being acquired, during one or more sleep cycles by one or more types of sensor;
processing, by the trained machine learning model, the sensor readings of the person and estimating, by the trained machine learning model, when the sensor readings indicate that the person is in one of sleep stage 3 (N3), sleep stage 4 (REM) or another sleep stage (which may be sleep stage 1 (N1) and/or sleep stage 2 (N2));
generating, by the trained machine learning model, control data based on the estimates of when the sensor readings indicate that the person is in one of at least sleep stage 3 (N3) and sleep stage 4 (REM) (and optionally sleep stage 1 (N1) and/or sleep stage 2 (N2)), the control data being tailored to the person for controlling a sleep enhancing device or system and an input provided thereby to act on the person for improving their sleep quality.

This aspect of the disclosure generates control data which are customised or tailored to a particular person's sleep pattern or sleep stages, for controlling a sleep enhancing device/system in an optimum way for that particular person. This can substantially improve their overall sleep quality by controlling operation of a sleep enhancing device/system in a way that complements the sleep stage that the person is in at various points during a sleep. Other advantages are similar to those discussed for the first aspect of the disclosure.

The processing step may include estimating, by the trained machine learning model, when the sensor readings indicate that the person enters one of sleep stage 3 (N3), sleep stage 4 (REM) or another sleep stage. The control data may include instructions to, when the person is estimated to have entered sleep stage 3 (N3) or sleep stage 4 (REM), stop or adjust the input provided by the sleep enhancing device or system.

This means that when the user is in a deep sleep, the movement of the bed can be reduced to a minimum or stopped. This can reduce possible adverse effects resulting from the sleep enhancing device or system, such as adverse effects from an oscillatory / rocking movement.

The processing step may include estimating, by the trained machine learning model, when the sensor readings indicate that sleep stage 3 (N3) and/or sleep stage 4 (REM) has elapsed by a predetermined extent. The control data may include instructions to, when the relevant sleep stage is estimated to have elapsed by the relevant predetermined extent, start or adjust the input provided by the sleep enhancing device or system.

For example, the instructions may lead to bed rocking at a predetermined speed or may increase the bed rocking speed. This can help to prolong sleep stage 3 and/or end sleep stage 4, relative to what would otherwise happen for the person during non-augmented sleep.

The one or more other sleep stages may include sleep stage 1 (N1) and/or sleep stage 2 (N2). The control data generated may be based on the estimates of when the sensor readings indicate that the person is in any one of sleep stage 3 (N3), sleep stage 4 (REM), and sleep stage 1 (N1) and/or sleep stage 2 (N2).

The control data may include instructions for controlling a rocking or reciprocating motion of the sleep enhancing device or system, including one or more bed oscillatory or reciprocation speed values.

The instructions in the control data may correspond to various features of the first aspect of the disclosure.

The sensor readings may be acquired when the person was or is asleep in an oscillating or reciprocating bed.

The data set may include a plurality of bed oscillatory or reciprocation speed values corresponding to each one of the sensor readings at the time that each one of the respective sensor readings was taken.

This allows data from a rocking bed to be taken into account when determining how a person's sleep stages change during sleep in a rocking bed as opposed to a conventional non-rocking bed.

The sensor readings may be from any one, two or more, three or more, four or more, five or more, or six or more of the following types of sensor: motion sensor, body temperature sensor, heart rate or pulse sensor, blood pressure sensor, EEG sensor, breathing rate sensor, ECG sensor, heart rate variability sensor.

As discussed earlier, using data from multiple sensors allows for greater accuracy when estimating which sleep stage a person is in at various points during their sleep.

If training the machine learning model *ab initio,* prior to processing a specific person's sleep data, then this may involve any one, some or all of:
receiving a data set (which may have been cleaned up and/or normalised and/or windowed) comprising a plurality of sensor readings acquired during one or more sleep cycles by one or more types of sensor (optionally selected from those set out above or in earlier aspects of the invention or disclosure, and
optionally including one or more polysomnograms), and optionally a plurality of input readings taken at times corresponding respectively to each of the plurality of sensor readings, each input reading corresponding to an input acting on the person at the time of the sensor reading (where the input may be a bed rocking speed);
receiving a sleep stage label for each sensor reading, including a sleep stage 3 (N3) label associated with each of the sensor readings acquired during sleep stage 3 (N3) of a person's sleep, and a sleep stage 4 (REM) label associated with each of the sensor readings acquired during sleep stage 4 (REM) of a person's sleep, and optionally corresponding labels for readings taken in sleep stage 1 (N1) and/or sleep stage 2 (N2);
optionally, if sensor readings from multiple types of sensor are provided, using a common timestamp or matching timestamps for the sensor readings;
optionally, receiving information concerning sensor reading value ranges and the value range correspondence to the respective sleep stages (e.g. by supervised learning);
training a machine learning model to identify at least sensor readings corresponding to sleep stage 3 (N3) and sensor readings corresponding to sleep stage 4 (REM) (and optionally sensor readings corresponding to either or both of sleep stage 1 (N1) and sleep stage 2 (N2)) using: each one of the plurality of sensor readings, the sleep stage label associated with each sensor reading, and optionally each one of the input readings taken at times corresponding respectively to each of the plurality of sensor readings, and optionally any of the other features above; which may include training using any one, some or all of a clustering module, a case-based reasoning module, a data mining module, a LSTM (Long Short-Term Memory) neural network module, where each of these modules is a known or publicly available module;
optionally wherein the training step involves training the machine learning model to make an estimation or determination of the sleep stage in which a given sensor reading is taken based on sensor reading values from two or more different types of sensor (unsupervised learning).

Note that supervised learning occurs by feeding previously captured data from sensors and the desired output bed movement control data to the algorithm, with the aim of training the model to yield the desired output; whilst unsupervised learning through which the algorithms learn from scratch and decide which is the best solution to control the bed movement, given a subject specific sleep peculiarities that might not fit into the previously known patterns or clusters

According to a fourth aspect of the present invention, there is provided a non-transitory computer readable medium containing program instructions for causing a processing device to perform a method of controlling a sleep enhancing device or system for improving sleep quality, as set out in claim 11. Optional features are set out in the dependent claims.

The advantages are similar to the first and second aspects.

According to a fifth aspect of the present disclosure, there is provided a non-transitory computer readable medium containing program instructions for causing a processing device to perform a method of generating, by a machine learning model, control data for controlling a sleep enhancing device or system for improving sleep quality, the instructions including:
instructions for providing or accessing a machine learning model trained to estimate a sleep stage at which a sensor reading was acquired including when a sensor reading was acquired during sleep stage (N3) and when a sensor reading was acquired during sleep stage 4 (REM) (and optionally when a sensor reading was acquired during sleep stage 1 (N1) and/or when a sensor reading was acquired during sleep stage 2 (N2)), or training such a machine learning model;
instructions for providing, to the trained machine learning model, a data set of a plurality of sensor readings of a person which have been acquired, or are being acquired, during one or more sleep cycles by one or more types of sensor;
instructions for processing, by the trained machine learning model, the sensor readings of the person and estimating, by the trained machine learning model, when the sensor readings indicate that the person is in one of sleep stage 3 (N3), sleep stage 4 (REM) or another sleep stage (optionally sleep stage 1 (N1) and/or sleep stage 2 (N2));
instructions for generating, by the trained machine learning model, control data based on the estimates of when the sensor readings indicate that the person is in one of at least sleep stage 3 (N3) and sleep stage 4 (REM) (and optionally sleep stage 1 (N1) and/or sleep stage 2 (N2)), the control data being tailored to the person for controlling a sleep enhancing device or system and an input provided thereby to act on the person for improving their sleep quality.

The advantages are similar to the fourth aspect of the invention.

A sleep enhancing device or system (such as a bed rocking device or a rocking chair or a motion imparting leg(s) for a bed) may be provided which comprises the non-transitory computer readable medium of the fourth or fifth aspects.

Any aspect of the invention or disclosure may include any feature or independently selected combination of features presented with respect to any other aspect of the invention or disclosure.

### BRIEF DESCRIPTION OF THE DRAWINGS

For a better understanding of the present invention, and to show more clearly how it may be carried into effect, reference will now be made by way of example only to the accompanying drawings, in which:
Figure 1 shows a perspective view of a person lying in a bed including a system for rocking the bed;
Figure 2 shows a flow chart exemplifying how data related to a person sleeping in a bed can be processed to provide bed motion control data which can be used in a predictive model for how to control rocking motion of the bed; and
Figure 3 shows another flow chart exemplifying how bed motion control data can be used for bed rocking according to predicted sleep stage.

### DESCRIPTION OF PREFERRED EMBODIMENTS

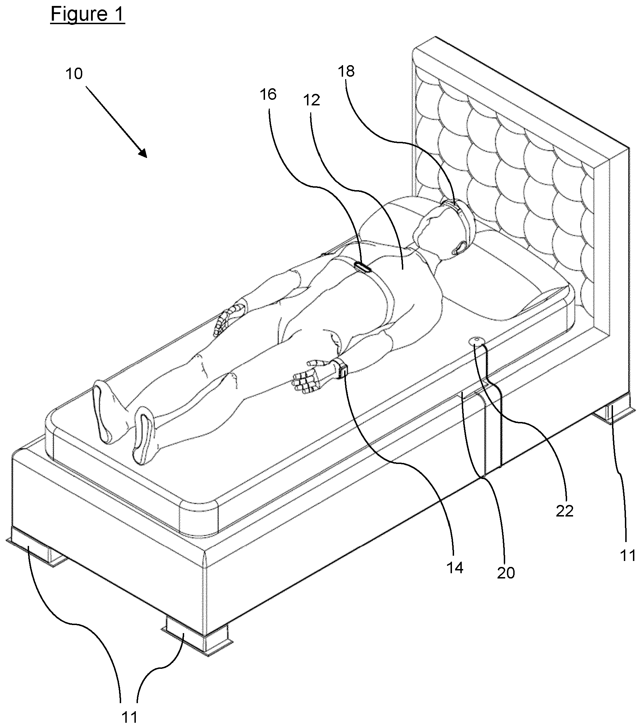

Referring firstly to Figure 1, a bed is indicated generally at 10. A person 12 is shown lying in the bed 10. The bed 10 has a mattress, a pillow, a headboard and a base.

In this embodiment, four bed legs 11 are provided under the four corners of the base. The bed legs 11 are motion imparting legs that can impart a reciprocating lateral rocking motion to the bed 10 so that it rocks back and forth in a horizontal plane when the legs are operating. Examples of the type of leg that can achieve this are presented in the Applicant's patent application WO2018083153 or the Applicant's patent application EP4137008.

The legs 11 can be interconnected (e.g. wirelessly) and preferably operate in a synchronised manner, so that the legs 11 at the left side of the bed 10 work antagonistically with the legs 11 on the right side of the bed 10 in rocking the bed. During use, when the legs 11 are turned on, the bed 10 can be displaced laterally left and right in a reciprocating manner within the limits of displacement provided by the legs.

The bed arrangement depicted has five different types of sensor device for sensing various parameters related to the person 12, for use in estimating which stage of sleep they are in at a given time during a sleeping episode. The sensor devices in this embodiment include: a wristwatch or bracelet 14, a chest belt 16, a headband 18, a mat sensor 20, and an accelerometer 22. It will be appreciated that the form of each type of device is not limited only to the depicted versions.

The bracelet or wristwatch 14 can have sensors for any of motion, temperature, measure heart rate / pulse, electrocardiogram (ECG), and blood pressure. The chest belt 16 can have sensors for any of movement, temperature, heart rate/pulse measurement, electrocardiogram (ECG), and heart rate variability (which can be derived from ECG). The head band 18 can have sensors for any of motion, temperature, and EEG (electro encephalogram). The mat-type sensor 20 can have sensors for any of motion, breathing rate, heart rate / pulse, and heart rate variability. It will be appreciated that any one, two, three or four, or all five, sensor types of the sensor devices may be used in any embodiment, but that it is preferred to use multiple sensors in some cases. It will also be appreciated that some of the sensor devices could be provided in a combined device, where a given sensor device has sensors common to two of the different ones illustrated. Examples of other sensor devices include a smartphone and a pulse oximeter.

An arrangement of this type can be used for a number of different people to record sleep data covering a wide range of body parameters during a series of sleep cycles.

In respect of supervised training, the following tables show the mapping of the information that is derived from each type of sensor in relation to each of the sleep stages (N1, N2, N3, REM). However, the ECG and heart rate variability sensors currently require specialised analysis algorithms (optionally using AI) to interpret how the sensor reading corresponds to sleep stage and so are not summarised below.

**Table I - Correspondence between sleep stage and relative expected sensor reading value for motion sensor, body temperature sensor and heart rate / pulse sensor**

| **Sleep stage** | **Motion sensor** | **Body temperature sensor** | **Heart rate / pulse sensor** |
|---|---|---|---|
| Stage 1 | high activity | temperature drops slightly (relative to being awake) | heart rate starts to drop slightly (relative to being awake) |
| Stage 2 | moderate activity | temperature drops (relative to stage 1) | heart rate drops (relative to stage 1) |
| Stage 3 | very low activity | temperature drops (relative to stage 2) | heart rate drops (relative to stage 2) |
| Stage 4 | very low activity | temperature raises (relative to stages 1, 2 and 3) | heart rate rises to levels that are similar to being awake |

**Table II - Correspondence between sleep stage and relative expected sensor reading value for blood pressure sensor, EEG sensor and breathing rate sensor**

| **Sleep stage** | **Blood pressure sensor** | **EEG sensor** | **Breathing rate sensor** |
|---|---|---|---|
| Stage 1 | it drops (relative to being awake) | more than 50% of the alpha waves are replaced with low-amplitude mixed-frequency waves (LAMF, 4-7 Hz) | it slows down (relative to being awake) |
| Stage 2 | it drops (relative to stage 1) | low-amplitude mixed-frequency wave (LAMF, 12-14 Hz) | it slows down slightly (relative to stage 1) |
| Stage 3 | it drops (relative to stage 2) | delta waves - lowest frequency, highest amplitude | it slows down slightly (relative to stage 2) |
| Stage 4 | it increases to levels that are similar to being awake | beta waves - similar to brain waves during wakefulness | faster and more erratic (relative to stages 1, 2 and 3) |

That is, in this case, the Al algorithm is told which sleep stage is attributed to each sensor reading for those sensors in the tables.

Note that there may be a step in which a doctor or a Smart Al software (which can analyse the sleep data e.g. from a whole night) provides or determines sleep stage information or decisions in respect of ECG and/or heart rate variability sensor readings. Afterwards, the ECG and/or heart rate variability data and the corresponding sleep stage for each epoch (30 or 60 seconds) can be fed to the Al algorithm for supervised training.

After collecting sensor data from a suitable sample size of different people, the data can be used as part of training a machine learning model to estimate which sleep stage a sleeping person is in based on sensor readings from one or more sensors.

In the case of supervised learning, the machine learning model may be provided with the sensor data and corresponding sleep stage information (e.g. derived from the sensor data or determined by a doctor). The sensor data may be replaced or supplemented by polysomnogram data. This is advantageous because the sleep stage information derived from a polysomnogram is much more accurate, thus improving the training of the machine learning model.

In the case of unsupervised learning, the machine learning model may be trained without having sleep stage information inputted, so that the output of the model / Al algorithm is the determination or estimation of the sleep stage attributable to each sensor reading. However, this is currently less accurate than sleep stage information determined by other means noted above, and so supervised learning for the machine learning model can result in better outcomes.

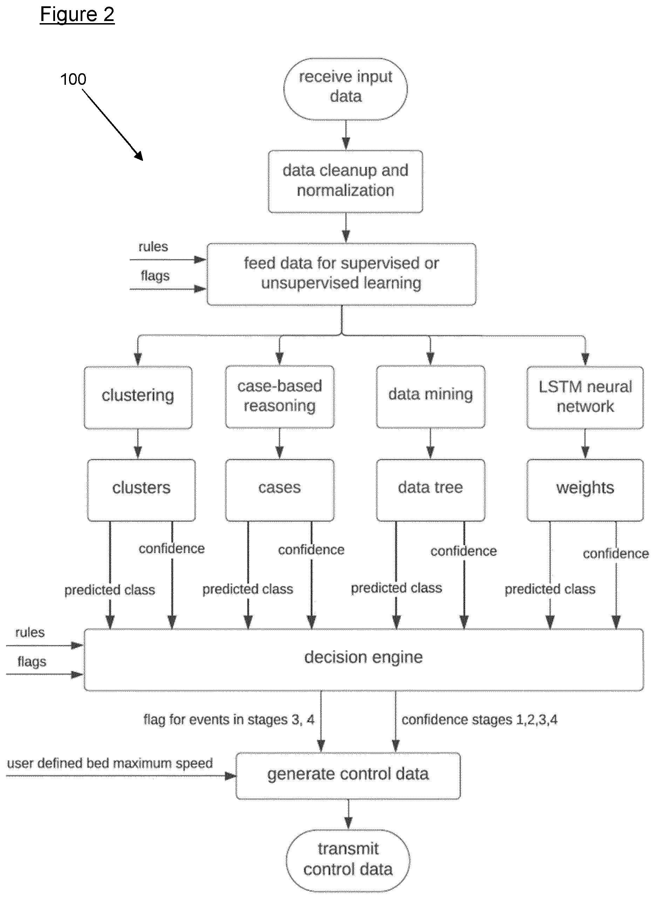

Figure 2 depicts a flow chart 100 of the general steps required for this process. The chart 100 corresponds to a proposed predictive model learning method that implements artificial intelligence and machine learning algorithms. The method generates predictive models from the input data that is fetched from sensors (optionally along with instantaneous bed oscillatory speed information), and produces, as an output, the control data which sets a bed rocking motion speed value.

In preparing the collected data for this purpose, the instantaneous sleep stage of the analysed subject in each case can be accurately estimated by using known methods in somnology (i.e. by a medically trained professional / doctor, or by smart Al software - preferably utilising polysomnogram data), in order to derive the sleep stage level starting from the specific and particular information provided by each type of sensor.

It will be appreciated that Al algorithms are able to undergo:
▪ supervised learning, which in this case can be done by feeding in previously captured data from sensors and the desired output control data (i.e. how to control the bed movement by the legs 11) to the algorithm, with the aim of training the model to yield the desired output; and/or
▪ unsupervised learning, through which the algorithms learn from scratch and decide which is the best solution to control the bed movement, given a subject specific sleep peculiarities that might not fit into the previously known patterns or clusters.

It is of course appreciated that the quality of a training data set is very important in order to accurately train an Al algorithm. Thus, as mentioned above, in some preferred embodiments it may be advantageous to obtain/use a polysomnogram for use in the case of supervised training, along with data from any of the above sensors. A polysomnogram very accurately captures the vital parameters of a person's body, and deriving sleep stage information based on the polysomnogram (and not from the other sensors above) can result in a more accurately trained model.

It will also be appreciated that some sensor readings / signals, such as EEG and ECG, could be pre-processed and/or transformed, for example into a frequency domain, with the aim of improving the accuracy of a model (once trained on the data). Automatic sleep stage classification by analysing EEG and ECG signals may be beneficial as part of the process, if available.

In some embodiments, the sleep stage at which the various sensor readings were recorded could be determined automatically by a software program, or determined manually by a trained specialist (such as a doctor).

Once the sensor readings have been correctly categorised according to the sleep stage that they correspond to, the sensor readings and the sleep stage information could be fed to the Al algorithms for supervised training.

However, it can be beneficial to filter, clean up, window, down sample and/or normalise the data first. The data clean-up and normalisation module indicated in the flowchart 100 has the role of translating / adapting all the sources of data from the various sensors and the bed oscillatory motion speed to:
a) a common sampling frequency that is specific to each type of captured vital parameter; and
b) a common data range, such as normalization to a common range that is specific to each type of sensor.

For (a), EEG and ECG sensors may have a sampling frequency (i.e. rate of taking readings) in the range 200 Hz to 500 Hz. Sensors for motion, body temperature, heart rate / pulse, heart rate variability, breathing rate and/or bed oscillatory motion speed may have a sampling frequency of about 1Hz.

For (b), EEG, ECG and/or motion sensor readings may be the range -1.0 to +1.0. Sensor readings for heart rate / pulse, heart rate variability and/or breathing rate may be in the range 0 to 256. Sensor readings for body temperature may be in the range 35 to 41 degrees Celsius or 95 to 106 degrees Fahrenheit. Sensor readings for bed oscillatory motion speed may be in the range 0 to 100 (which may correlate to: 0 to about 10 mm per second).

The data clean-up and normalisation module may use:
- one or more low pass filters for smoothing the sensor data, and/or
- an anomaly prediction algorithm which filters out noisy or erroneous or unwanted data based on the known characteristics of the previously received data.

This can result in cleaner dataset that can in turn lead to more accurate estimations / predictions from the model (once trained).

The data clean-up and module can also handle windowing of the incoming data into a standard length set (e.g. processing frame size). This may correspond to 30 seconds or 60 seconds, for example, although the window size value could be set to another value instead. In view of the large quantity of data accumulated over time by running an Al algorithm, this may reduce the system large memory requirements by down sampling data that carries redundant information.

The next module represents a person (e.g. supervisor or Al software engineer) who classifies data for supervised or unsupervised processing by setting appropriate flags. This is done for producing training, test, blind and run time data sets, which are respectively used for the creation, testing, verification/validation and real time run of the machine learning model / algorithm. Each of these is a well known step for building data sets for a machine learning model.

The control rules for the classification module are:
- type of input data: training, test, blind, verification;
- in the case of supervised training, provide the known sleep stage information to the trained algorithm.

The control flags for the classification module are:
- supervised or unsupervised;
- first time user, or not.

Each of the modules immediately after the classification module is a well-known type of Al algorithm which can predict with a certain confidence and satisfies the requirements for training a machine learning model on the sleep data set: clustering, case-based reasoning, data mining, LSTM (Long Short-Term Memory) neural network.

The clustering module is capable of user sleep classification into clusters which cover sleep disorders/dysfunctions and normal types of sleep behaviour.

The case-based reasoning module is capable of addressing new problems by retrieving stored 'cases' describing similar prior problem-solving episodes and adapting the solutions to fit the new needs.

The data mining module is capable of extracting, deriving or abstracting rules from large quantities of data.

The LSTM neural network is capable of long-term and short-term sleep stage prediction.

A decision engine then needs to select the most relevant information from each of the four above Al modules. Along with user set control rules and flags, it then produces the sleep stage confidence levels / thresholds and the flags for when to start, stop or adjust the input (e.g. bed motion) in sleep stage 3 and/or sleep stage 4, and optionally sleep stage 1 and/or sleep stage 2.

Note that in sleep stage 1 and/or sleep stage 2, the input may be a user-selected preset which has a non-zero value. For example, for a sleep enhancing device including a motion imparting device for reciprocating rocking of the bed, a non-zero speed for bed reciprocation may be used.

Typically, the flag or trigger upon which to initiate or "switch on" motion for sleep stage 3 will be set at a few minutes before the predicted lapsing time of sleep stage 3. The exact point at which this is set is based on the deemed success rate of previous starts. That is, if in a previous sleep stage 3 the input was set at time *t-5* mins from the predicted end time *t* of a sleep stage 3, but the user in fact transitioned out of the sleep stage 3 earlier than predicted, then in a subsequent sleep stage 3 the input will be scheduled to start suitably earlier to mitigate the likelihood of starting too late again.

Typically, the flag or trigger upon which to initiate or "switch on" motion for sleep stage 4 will be set at about half of the usual time that is spent in sleep stage 4, i.e. at around the midway point of sleep stage 4. The REM period duration is different for each sleep cycle, and increases throughout the night. For example, there is a shorter REM sleep period for the first sleep cycle and relatively longer REM sleep period for the later sleep cycles. This means that the length of time for which bed leg motion is ceased for sleep stage 4 may need to be adjusted over several nights, so that the Al algorithm can learn the person's specific REM sleep duration for each of their sleep cycles.

The control rules for the decision engine are:
- Desired total length of sleep time.
   ∘ This can be set by the user via a remote control for the bed legs 11 or via an app which is in remote communication with the bed legs 11.
   ∘ This information is used for switching off the bed motion during the last sleep cycle, thus avoiding the unwanted effects of getting dizzy when waking up.
- Any previously clinically diagnosed sleep condition / disorder which would classify the user into a certain cluster.
   o Here, the Al generated cluster information is overridden by the clinical diagnosis, i.e. that provided by a doctor.
- Optionally, a predefined time (set by the user or a medical professional) for turning on the bed oscillatory motion before the end of the sleep stage 3.
- Optionally, a predefined time (set by the user or a medical professional) for turning on the bed oscillatory motion after the onset of the sleep stage 4.

The control flags for the decision engine are:
- day time nap, or night time sleep;
- first time user, or not.

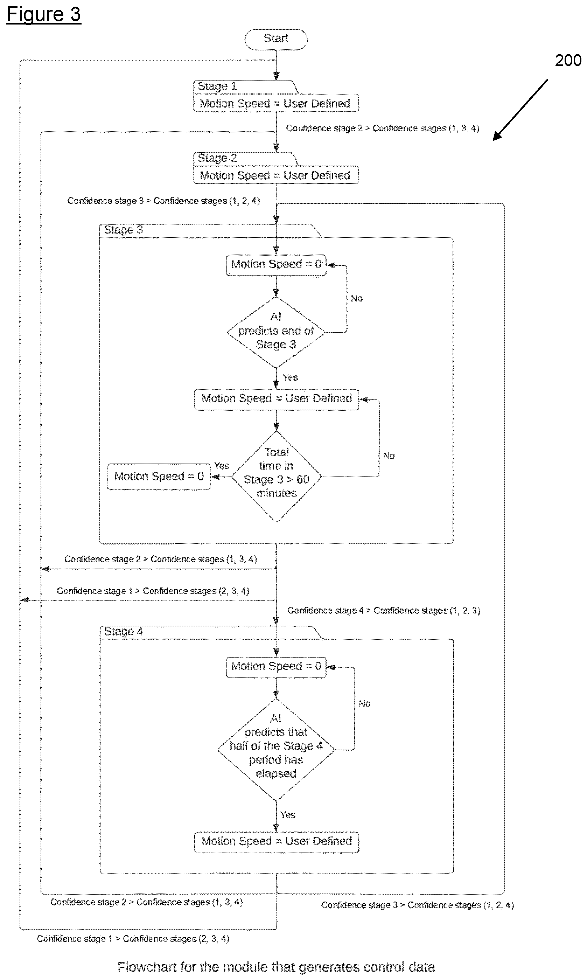

Figure 3 (flow chart 200) and Table III set out the behaviour of the generation module that generates the bed motion control data. For each sleep stage there is a target bed oscillatory speed that is derived from the run time estimated sleep stage confidence.

**Table III - Example bed oscillation / reciprocation speed values and modulation thereof according to sleep stage**

| **Sleep stage** | **Bed oscillatory/reciprocating speed criteria** |
|---|---|
| Stage 1 | Set by the user to a value between 0 and 100% |
| Stage 2 | Set by the user to a value between 0 and 100% |
| Stage 3 | Fade out the bed motion speed to 0% (stop) after the onset of sleep stage 3. Keep it stopped until the motion "switch on" flag for the sleep stage 3 is triggered by the decision engine. |
| | After the "switch on" flag is triggered, the bed oscillatory speed can be gradually increased or ramped up to the person's pre-selected bed speed (set by app or remote control). |
| | If the selected speed is not effective enough to keep the user in sleep stage 3, the speed is augmented with an increment of 5% and used at that value for sleep stage 3 in subsequent sleep cycles. |
| | Fade out the bed motion speed to 0% (stop) if the sleep stage 3 duration for the current sleep stage 3 exceeds 60 minutes. |
| Stage 4 | Fade out the bed motion speed to 0% (Stop) after the onset of sleep stage 4. |
| | Keep it stopped until the motion "switch on" flag for sleep stage 4 is triggered by the decision engine. |
| | If this bed speed is not effective enough for taking the user out of sleep stage 4, the speed is augmented with an increment of 5% and used at that value for sleep stage 4 in subsequent sleep cycles. |

Having trained a machine learning model broadly as indicated above, or otherwise having access to a suitably trained model, a sleep data set acquired using some or all of the sensors or sensor devices (discussed with respect to Figure 1) can be processed or analysed by the trained model. This may be done for the first time by having the Al model estimate sleep stage as the person 12 sleeps, or the Al model may be provided with data corresponding to one or several nights worth of sleep for the person 12.

The Al model can then, according to its training, take the sensor readings from the available sensors and assess the likelihood that the person 12 is/was in a state corresponding to sleep stage 1 (N1), sleep stage 2 (N2), sleep stage 3 (N3), or sleep stage 4 (REM), and can provide a confidence stage/level for each of these. The confidence levels for all four sleep stages (that is, the probability that a particular sleep stage is the 'active' sleep stage at a given time) must always sum to 100%. It should be noted that only one sleep stage may be active at a time, but there might be edge cases when two of them are assigned equally large confidence levels.

The Al model can also, according to its training, take the sensor readings from the available sensors and identify markers or indications in the data that signal the onset and end of the various sleep stages, and in particular: markers or indications in the data that signal the onset of sleep stage 3, markers or indications in the data that signal the onset of sleep stage 4, markers or indications in the data that signal the end of sleep stage 3, and markers or indications in the data that signal the end of sleep stage 4. This allows for estimation of the length of each of that person's sleep stages, accounting for variability in the length of the sleep stages as sleep cycles progress.

Control data can be generated by the trained machine learning model based on the data corresponding to the person 12. The control data include instructions concerning when to start an input and/or when to stop an input and/or when to adjust (increase/decrease/change) an input, which in this case is a motion speed imparted by the bed legs 11.

In particular, the control data can start/stop/change an input at the onset of sleep stage 3, at the onset of sleep stage 4, near the end of sleep stage 3, and/or around the middle of sleep stage 4, although other points may additionally or alternatively be selected for taking action regarding the input. Example criteria for starting/stopping/changing the input are discussed above with respect to Figure 3 and Table III.

The control data thus prepared can be used to control the input provided by the bed legs 11 each time that the person 12 sleeps on the bed 10, so that the bed legs 11 rock the bed 10 in a way that improves that person's sleep cycles and relative abundance of sleep stages within those cycles. In particular, the control data is intended to improve the duration of sleep stage 3 (N3) for the person when they sleep and to relatively controllably alternate between sleep stage 3 (N3) and sleep stage 4 (REM) for the person 12 when they sleep.

It will be appreciated that, in implementing the present invention, the algorithm(s) could run locally on a processor of the rocking bed 10 (or the legs 11 thereof), or on a server located on the internet (e.g. in the cloud) which could include bidirectional data communication and control between the moving bed 10 and the cloud.

If using a cloud configuration, captured real-time data from sensors could be sent to the cloud optionally along with the bed's instantaneous oscillatory movement parameters (e.g. speed), and in the other direction real-time commands/instructions with the bed's motion parameters (e.g. bed oscillatory motion speed information) can be sent from the cloud to the moving bed 10.

It is possible for the sensors to be connected directly to the rocking bed (e.g. to the processor) or the sensors to communicate through other methods (e.g. the internet) with the cloud in which the artificial intelligence algorithms could run.

A hardware platform that support algorithms for neural network computation may be used to implement the invention. The functional blocks of the method may be independently operated on a microservice platform.

It will be appreciated that the invention has potential applications to provide better sleep in various different scenarios. This includes scenarios beyond sleeping in a bed. For example, implementing the invention in a seat in a vehicle such a plane or train or boat may help improve sleep quality for a passenger.

The embodiments described above are provided by way of example only, and various changes and modifications will be apparent to persons skilled in the art without departing from the scope of the present invention as defined by the appended claims.

## Claims

1. A system for improving sleep quality for a sleeping person (12), the system comprising:
one or more sleep enhancing devices which include one or more bed rocking devices (11) or systems configured to provide a bed rocking or reciprocating motion as an input for acting on the person (12);
one or more sensors (12, 14, 16, 18, 20, 22) for positioning on or near the person (12), each of the one or more sensors (12, 14, 16, 18, 20, 22) being selected from the following group: motion sensor, accelerometer, temperature sensor, breathing rate sensor, electrocardiogram (ECG) sensor, heart rate sensor, pulse sensor, heart rate variability sensor, blood pressure sensor such as blood arterial pressure sensor, electro encephalogram (EEG) sensor;
bed motion control data including instructions or flags concerning one, some or all of: when to start the bed rocking or reciprocating motion, when to stop the bed rocking or reciprocating motion and when to adjust the bed rocking or reciprocating motion, the bed motion control data including one or both of:
a first instruction or flag corresponding to a predicted end of sleep stage 3 (N3) for the person, and
a second instruction or flag corresponding to a predicted middle of sleep stage 4 (REM) for the person; and
a processing device configured to:
receive information from the one or more sensors (12, 14, 16, 18, 20, 22),
use the received information to generate or obtain an estimate about which stage of sleep the person (12) is in, the stage of sleep being selected from a group comprising: sleep stage 3 (N3), sleep stage 4 (REM) and one or more other sleep stages; and
based on the bed motion control data, control the one or more sleep enhancing devices including starting, stopping or changing the input provided thereby when the person (12) is estimated to be in sleep stage 3 (N3) and/or sleep stage 4 (REM), including one or both of:
based on the first instruction or flag, start the bed rocking or reciprocating motion near the predicted end of sleep stage 3 (N3), and
based on the second instruction or flag, start the bed rocking or reciprocating motion at around the predicted middle of sleep stage 4 (REM).

2. A system as claimed in claim 1, comprising a trained machine learning model configured to generate the bed motion control data based on data corresponding to the person (12).

3. A system as claimed in claim 2, in which the processing device is configured to:
estimate, by the trained machine learning model, based on information from at least the one or more sensors (12, 14, 16, 18, 20, 22), when the person (12) enters sleep stage 3 (N3) and/or sleep stage 4 (REM); and
when the person (12) is estimated to have entered sleep stage 3 (N3) or sleep stage 4 (REM), stop or adjust the bed rocking or reciprocating motion provided by the one or more sleep enhancing devices.

4. A system as claimed in any of claims 1 to 3, in which the first instruction or flag corresponds to up to around 10 minutes before the predicted end of that sleep stage 3 (N3); optionally in which, where the person is estimated to be in sleep stage 3 (N3), the processing device is configured to stop the bed rocking or reciprocating motion started based on the first instruction or flag if the duration of that sleep stage 3 (N3) exceeds about 60 minutes or exceeds a predetermined length of time.

5. A system as claimed in any preceding claim, in which the one or more sleep enhancing devices are programmable with a first predetermined input value for sleep stage 3 (N3), and the bed rocking or reciprocating motion is set at the first predetermined input value near the predicted end of the sleep stage 3 (N3).

6. A system as claimed in claim 5, in which if the first predetermined input value is insufficient to prolong a first sleep stage 3 (N3) period, the system is configured to use a higher input value for the bed rocking or reciprocating motion during one or more subsequent sleep stage 3 (N3) periods.

7. A system as claimed in any preceding claim, in which the one or more sleep enhancing devices are programmable with a second predetermined input value for sleep stage 4 (REM), and the bed rocking or reciprocating motion is set at the second predetermined input value at the predicted middle of the sleep stage 4 (REM),

8. A system as claimed in claim 7, in which if the second predetermined input value is insufficient to end a first sleep stage 4 (REM) period early, the system is configured to use a higher input value for the bed rocking or reciprocating motion during one or more subsequent sleep stage 4 (REM) periods.

9. A system as claimed in any preceding claim, in which the one or more sleep enhancing devices are programmable with one or both of:
clinical information for the person (12), and any one or more of the following input parameters are controlled based at least in part on the clinical information: input type, input intensity, input start time, input end time, input duration, input pattern; and
a sleep duration value which is measured beginning from a clock time or a time when the person is detected as having fallen asleep, and where a given sleep stage 3 (N3) or sleep stage 4 (REM) is predicted to be the last sleep stage 3 (N3) or last sleep stage 4 (REM) before the sleep duration value is exceeded, the one or more sleep enhancing devices and the associated input are stopped and do not restart when the person enters any subsequent sleep stage before they awake.

10. A system as claimed in any preceding claim, using a cloud configuration, in which captured real-time data from the one or more sensors is sent to the cloud optionally along with speed or other instantaneous oscillatory movement parameters of the bed (10), and real-time commands/instructions with motion parameters of the bed, such as bed oscillatory motion speed information, are sent from the cloud to the moving bed (10).

11. A non-transitory computer readable medium containing program instructions for causing a processing device to perform a method of controlling a sleep enhancing device or system which is a bed rocking device or system for improving sleep quality, the instructions including:
instructions for estimating, based on information from at least one or more sensors (12, 14, 16, 18, 20, 22), which stage of sleep a person (12) is in, the stage of sleep being selected from a group comprising: sleep stage 3 (N3), sleep stage 4 (REM) and one or more other sleep stages; and
bed motion control data including instructions or flags concerning one, some or all of: when to start a bed rocking or reciprocating motion, when to stop a bed rocking or reciprocating motion and when to adjust a bed rocking or reciprocating motion, the bed motion control data including one or both of:
a first instruction or flag corresponding to a predicted end of sleep stage 3 (N3) for the person, and
a second instruction or flag corresponding to a predicted middle of sleep stage 4 (REM) for the person;
instructions for, when the person (12) is estimated to be in sleep stage 3 (N3) and/or when the person (12) is estimated to be in sleep stage 4 (REM), performing one or both of the following based on the bed motion control data:
based on the first instruction or flag, starting the sleep enhancing device or system near the predicted end of sleep stage 3 (N3) to begin providing a bed rocking or reciprocating motion as an input which acts on the person (12); and
based on the second instruction or flag, starting the sleep enhancing device or system near the predicted middle of sleep stage 4 (REM) to begin providing a bed rocking or reciprocating motion as an input which acts on the person (12).

12. A non-transitory computer readable medium as claimed in claim 11, including a first predetermined input value for sleep stage 3 (N3), and the bed rocking or reciprocating motion is set at the first predetermined input value near the predicted end of the sleep stage 3 (N3).

13. A non-transitory computer readable medium as claimed in claim 12, in which if the first predetermined input value is insufficient to prolong a first sleep stage 3 (N3) period, a higher input value is used for the bed rocking or reciprocating motion during one or more subsequent sleep stage 3 (N3) periods.

14. A non-transitory computer readable medium as claimed in any of claims 11 to 13, including a second predetermined input value for sleep stage 4 (REM), and the bed rocking or reciprocating motion is set at the second predetermined input value at the predicted middle of the sleep stage 4 (REM).

15. A non-transitory computer readable medium as claimed in claim 14, in which if the second predetermined input value is insufficient to end a first sleep stage 4 (REM) period early, a higher input value is used for the bed rocking or reciprocating motion during one or more subsequent sleep stage 4 (REM) periods.

## Patentansprüche

1. System zur Verbesserung der Schlafqualität für eine schlafende Person (12), wobei das System umfasst:
ein oder mehrere schlaffördernde Vorrichtungen, die ein oder mehrere Bettschaukelvorrichtungen (11) oder Systeme enthalten, die konfiguriert sind, um eine Schaukel-, oder Hin- und Herbewegung des Bettes als Eingabe zum Einwirken auf die Person (12) bereitzustellen;
einen oder mehrere Sensoren (12, 14, 16, 18, 20, 22) zum Positionieren auf oder in der Nähe der Person (12), wobei jeder des einen oder mehrerer Sensoren (12, 14, 16, 18, 20, 22) aus der folgenden Gruppe ausgewählt ist:
Bewegungssensor, Beschleunigungsmesser, Temperatursensor, Atemfrequenzsensor, Elektrokardiogramm-, (EKG)-Sensor, Herzfrequenzsensor, Pulssensor, Sensor für Herzfrequenzvariabilität, Blutdrucksensor wie arteriellem Blutdrucksensor, Elektroenzephalogramm-, (EEG)-Sensor;
Bettbewegungssteuerungsdaten, die Anweisungen oder Kennzeichnungen bezüglich eines, einiger oder aller von Folgenden enthalten: wann die Schaukel-, oder Hin- und Herbewegung des Bettes gestartet werden soll, wann die Schaukel-, oder Hin- und Herbewegung des Bettes gestoppt werden soll und wann die Schaukel-, oder Hin- und Herbewegung des Bettes angepasst werden soll, wobei die Bettbewegungssteuerungsdaten eines oder beide von Folgenden enthalten:
einer ersten Anweisung oder Kennzeichnung, die einem vorhergesagten Ende des Schlafstadiums 3 (N3) für die Person entspricht, und
einer zweiten Anweisung oder Kennzeichnung, die einer vorhergesagten Mitte des Schlafstadiums 4 (REM) für die Person entspricht; und
einer Verarbeitungsvorrichtung, die konfiguriert ist, um: Informationen von dem einen oder mehreren Sensoren (12, 14, 16, 18, 20, 22) zu empfangen,
die empfangenen Informationen zu verwenden, um eine Schätzung darüber zu erzeugen oder zu erhalten, in welchem Schlafstadium sich die Person (12) befindet, wobei das Schlafstadium aus einer Gruppe ausgewählt wird, umfassend: Schlafstadium 3 (N3), Schlafstadium 4 (REM) und eine oder mehrere andere Schlafstadien; und
basierend auf den Bettbewegungssteuerungsdaten die eine oder mehrere schlaffördernden Vorrichtungen, die Starten, Stoppen oder Ändern der Eingabe enthalten, zu steuern, die dadurch bereitgestellt wird, wenn geschätzt wird, dass die Person (12) im Schlafstadium 3 (N3) und/oder Schlafstadium 4 (REM) ist, eines oder beide von Folgenden enthalten:
basierend auf der ersten Anweisung oder Kennzeichnung, die Schaukel-, oder Hin- und Herbewegung des Bettes nahe dem vorhergesagten Ende des Schlafstadiums 3 (N3) zu starten, und
basierend auf der zweiten Anweisung oder Kennzeichnung die Schaukel-, oder Hin- und Herbewegung des Bettes etwa zur vorhergesagten Mitte des Schlafstadiums 4 (REM) zu starten.

2. System nach Anspruch 1, umfassend ein trainiertes maschinelles Lernmodell, das konfiguriert ist, um die Bettbewegungssteuerungsdaten basierend auf Daten, die der Person (12) entsprechen, zu erzeugen.

3. System nach Anspruch 2, wobei die Verarbeitungsvorrichtung konfiguriert ist, um:
durch das trainierte maschinelle Lernmodell basierend auf Informationen von mindestens dem einen oder mehreren Sensoren (12, 14, 16, 18, 20, 22) zu schätzen, wann die Person (12) ins Schlafstadium 3 (N3) und/oder Schlafstadium 4 (REM) eintritt; und
wenn geschätzt wird, dass die Person (12) ins Schlafstadium 3 (N3) oder Schlafstadium 4 (REM) eingetreten ist, die Schaukel-, oder Hin- und Herbewegung des Bettes, die von einer oder mehreren schlaffördernden Vorrichtungen bereitgestellt wird, zu stoppen oder anzupassen.

4. System nach einem der Ansprüche 1 bis 3, bei dem die erste Anweisung oder Kennzeichnung bis zu etwa 10 Minuten vor dem vorhergesagten Ende des Schlafstadiums 3 (N3) entspricht; wobei die Verarbeitungsvorrichtung optional, wenn geschätzt wird, dass die Person im Schlafstadium 3 (N3) ist, konfiguriert ist, die auf der ersten Anweisung oder Kennzeichnung basierende Schaukel-, oder Hin- und Herbewegung zu stoppen, wenn die Dauer dieses Schlafstadiums 3 (N3) etwa 60 Minuten überschreitet oder eine vorbestimmte Zeitdauer überschreitet.

5. System nach einem vorstehenden Anspruch, wobei die eine oder mehrere schlaffördernde Vorrichtungen mit einem ersten vorbestimmten Eingabewert für das Schlafstadium 3 (N3) programmierbar sind und die Schaukel-, oder Hin- und Herbewegung des Bettes auf den ersten vorbestimmten Eingabewert nahe dem vorhergesagten Ende des Schlafstadiums 3 (N3) gesetzt ist.

6. System nach Anspruch 5, wobei das System, falls der erste vorbestimmte Eingabewert nicht ausreicht, um ein erstes Schlafstadium 3 (N3) zu verlängern, konfiguriert ist, um einen höheren Eingabewert für die Schaukel-, oder die Hin- und Herbewegung des Bettes während einer oder mehrerer nachfolgender Schlafstadien N3 (N3) zu verwenden.

7. System nach einem vorstehenden Anspruch, wobei die eine oder mehrere schlaffördernden Vorrichtungen mit einem zweiten vorbestimmten Eingabewert für Schlafstadium 4 (REM) programmierbar sind und die Schaukel-, oder Hin- und Herbewegung des Bettes auf den zweiten vorbestimmten Eingabewert in der vorhergesagten Mitte des Schlafstadiums 4 (REM) gesetzt ist,

8. System nach Anspruch 7, wobei das System, falls der zweite vorbestimmte Eingabewert nicht ausreicht, um ein erstes Schlafstadium 4 (REM) vorzeitig zu beenden, konfiguriert ist, um während einer oder mehrerer nachfolgender Schlafstadien 4 (REM) einen höheren Eingabewert für die Schaukel-, oder Hin- und Herbewegung des Bettes zu verwenden.

9. System nach einem vorstehenden Anspruch, wobei die eine oder mehrere schlaffördernde Vorrichtungen mit einem oder beiden programmierbar sind, von:
klinischen Informationenen für die Person (12), und ein oder mehrere der folgenden Eingabeparameter mindestens teilweise basierend auf den klinischen Informationen gesteuert werden: Eingabetyp, Eingabeintensität, Eingabestartzeit, Eingabeendzeit, Eingabedauer, Eingabemuster; und
ein Schlafdauerwert, der beginnend mit einer Uhrzeit oder einem Zeitpunkt gemessen wird, an dem erkannt wird, dass die Person eingeschlafen ist, und bei dem ein gegebenes Schlafstadium 3 (N3) oder Schlafstadium 4 (REM) als das letzte Schlafstadium 3 (N3) oder letzte Schlafstadium 4 (REM) vorhergesagt wird, bevor der Schlafdauerwert überschritten wird, werden, die eine oder mehrere schlaffördernde Vorrichtungen und die zugehörige Eingabe gestoppt wird und nicht erneut startet, wenn die Person vor dem Aufwachen in ein nachfolgendes Schlafstadium eintritt.

10. System nach einem vorstehenden Anspruch, das eine Cloud-Konfiguration verwendet, bei der erfasste Echtzeitdaten von dem einen oder mehreren Sensoren optional zusammen mit der Geschwindigkeit oder anderen momentanen oszillatorischen Bewegungsparametern des Bettes (10) in die Cloud gesendet werden, und Echtzeitbefehle/- anweisungen mit Bewegungsparametern des Bettes, wie z. B. Informationen zur oszillatorischen Bewegungsgeschwindigkeit des Bettes, von der Cloud an das sich bewegende Bett (10) gesendet werden.

11. Nichtflüchtiges, computerlesbares Medium, das Programmanweisungen zum Veranlassen einer Verarbeitungsvorrichtung enthält, ein Verfahren zur Steuerung einer schlaffördernden Vorrichtung oder eines Systems auszuführen, das eine Bettschaukelvorrichtung oder ein solches System zur Verbesserung der Schlafqualität ist, wobei die Anweisungen enthalten:
Anweisungen zum Schätzen, basierend auf Informationen von mindestens einem oder mehreren Sensoren (12, 14, 16, 18, 20, 22), in welchem Schlafstadium eine Person (12) ist, wobei das Schlafstadium aus einer Gruppe ausgewählt wird, umfassend: Schlafstadium 3 (N3), Schlafstadium 4 (REM) und eine oder mehrere andere Schlafstadien; und Bettbewegungssteuerungsdaten, die Anweisungen oder Kennzeichnungen bezüglich eines, einiger oder aller beinhalten von: wann eine Schaukel-, oder Hin- und Herbewegung des Bettes gestartet werden soll, wann eine Schaukel-, oder Hin- und Herbewegung des Bettes gestoppt werden soll und wann eine Schaukel-, oder Hin- und Herbewegung des Bettes angepasst werden soll, wobei die Bettbewegungssteuerungsdaten eines oder beide von Folgenden enthalten:
einer ersten Anweisung oder Kennzeichnung, die einem vorhergesagten Ende des Schlafstadiums 3 (N3) für die Person entspricht, und
einer zweiten Anweisung oder Kennzeichnung, die einer vorhergesagten Mitte des Schlafstadiums 4 (REM) für die Person entspricht;
Anweisungen zum, wenn geschätzt wird, dass die Person (12) im Schlafstadium 3 (N3) und/oder wenn geschätzt wird, dass die Person (12) im Schlafstadium 4 (REM) ist, Durchführen eines oder beider von Folgendem basierend auf den Bettbewegungssteuerungsdaten:
basierend auf der ersten Anweisung oder Kennzeichnung, Starten der schlaffördernden Vorrichtung oder Systems nahe dem vorhergesagten Ende des Schlafstadiums 3 (N3), um mit dem Bereitstellen einer Schaukel-, oder Hin- und Herbewegung des Bettes als Eingabe zu beginnen, die auf die Person (12) wirkt; und
basierend auf der zweiten Anweisung oder Kennzeichnung, Starten der schlaffördernden Vorrichtung oder Systems nahe der vorhergesagten Mitte des Schlafstadiums 4 (REM), um mit dem Bereitstellen einer Schaukel-, oder Hin- und Herbewegung des Bettes als Eingabe zu beginnen, die auf die Person (12) wirkt.

12. Nichtflüchtiges, computerlesbares Medium nach Anspruch 11, das einen ersten vorbestimmten Eingabewert für Schlafstadium 3 (N3) enthält, wobei die Schaukel-, oder Hin- und Herbewegung des Bettes auf den ersten vorbestimmten Eingabewert nahe dem vorhergesagten Ende des Schlafstadiums 3 (N3) gesetzt ist.

13. Nichtflüchtiges, computerlesbares Medium nach Anspruch 12, wobei, falls der erste vorbestimmte Eingabewert nicht ausreicht, um ein erstes Schlafstadium 3 (N3) zu verlängern, ein höherer Eingabewert für die Schaukel-, oder Hin- und Herbewegung des Bettes während einer oder mehrerer nachfolgender Schlafstadien N3 (N3) verwendet wird.

14. Nichtflüchtiges, computerlesbares Medium nach einem der Ansprüche 11 bis 13, das einen zweiten vorbestimmten Eingabewert für Schlafstadium 4 (REM) enthält, wobei die Schaukel-, oder Hin- und Herbewegung des Bettes auf den zweiten vorbestimmten Eingabewert in der vorhergesagten Mitte des Schlafstadiums 4 (REM) gesetzt ist.

15. Nichtflüchtiges, computerlesbares Medium nach Anspruch 14, wobei, falls der zweite vorbestimmte Eingabewert nicht ausreicht, um ein erstes Schlafstadium 4 (REM) vorzeitig zu beenden, ein höherer Eingabewert für die Schaukel-, oder Hin- und Herbewegung des Bettes während einer oder mehrerer nachfolgender Schlafstadien 4 (REM) verwendet wird.

## Revendications

1. Système pour améliorer la qualité du sommeil d'une personne (12) endormie, le système comprenant :
un ou plusieurs dispositifs améliorant le sommeil qui incluent un ou plusieurs dispositifs ou systèmes de balancement de lit (11) configurés pour fournir un mouvement de balancement ou de va-et-vient du lit en tant qu'entrée destinée à agir sur la personne (12) ;
un ou plusieurs capteurs (12, 14, 16, 18, 20, 22) destinés à être positionnés sur ou près de la personne (12), chacun des un ou plusieurs capteurs (12, 14, 16, 18, 20, 22) étant sélectionné dans le groupe suivant : capteur de mouvement, accéléromètre, capteur de température, capteur de fréquence respiratoire, capteur d'électrocardiogramme (ECG), capteur de fréquence cardiaque, capteur de pouls, capteur de variabilité de la fréquence cardiaque, capteur de pression artérielle tel qu'un tensiomètre, capteur d'électroencéphalogramme (EEG) ;
des données de commande de mouvement de lit incluant des instructions ou des indicateurs concernant un, certains ou la totalité des éléments suivants : quand démarrer le mouvement de balancement ou de va-et-vient du lit, quand arrêter le mouvement de balancement ou de va-et-vient du lit et quand ajuster le mouvement de balancement ou de va-et-vient du lit, les données de commande de mouvement de lit incluant l'un et/ou l'autre élément parmi :
une première instruction ou un premier indicateur correspondant à une fin prédite de la phase de sommeil 3 (N3) pour la personne, et
une seconde instruction ou un second indicateur correspondant à un milieu prédit de la phase de sommeil 4 (REM) pour la personne ; et
un dispositif de traitement configuré pour :
recevoir des informations provenant des un ou plusieurs capteurs (12, 14, 16, 18, 20, 22),
utiliser les informations reçues pour générer ou obtenir une estimation de la phase de sommeil dans laquelle se trouve la personne (12), la phase de sommeil étant sélectionnée dans un groupe comprenant : phase de sommeil 3 (N3), phase de sommeil 4 (REM) et une ou plusieurs autres phases de sommeil ; et
sur la base des données de commande de mouvement de lit, commander les un ou plusieurs dispositifs améliorant le sommeil, incluant le démarrage, l'arrêt ou la modification de l'entrée fournie par ceux-ci lorsqu'il est estimé que la personne (12) est en phase de sommeil 3 (N3) et/ou en phase de sommeil 4 (REM), incluant l'un et/ou l'autre parmi :
sur la base de la première instruction ou du premier indicateur, le démarrage du mouvement de balancement ou de va-et-vient du lit vers la fin prédite de la phase de sommeil 3 (N3), et
sur la base de la seconde instruction ou du second indicateur, le démarrage du mouvement de balancement ou de va-et-vient du lit aux alentours du milieu prédit de la phase de sommeil 4 (REM).

2. Système selon la revendication 1, comprenant un modèle d'apprentissage automatique formé configuré pour générer les données de commande de mouvement de lit sur la base de données correspondant à la personne (12).

3. Système selon la revendication 2, dans lequel le dispositif de traitement est configuré pour :
estimer, par le modèle d'apprentissage automatique formé, sur la base des informations provenant d'au moins les un ou plusieurs capteurs (12, 14, 16, 18, 20, 22), le moment où la personne (12) entre dans la phase de sommeil 3 (N3) et/ou la phase de sommeil 4 (REM) ; et
lorsqu'il est estimé que la personne (12) est entrée dans la phase de sommeil 3 (N3) ou la phase de sommeil 4 (REM), arrêter ou ajuster le mouvement de balancement ou de va-et-vient du lit fourni par les un ou plusieurs dispositifs améliorant le sommeil.

4. Système selon l'une quelconque des revendications 1 à 3, dans lequel la première instruction ou le premier indicateur correspond au maximum à environ 10 minutes avant la fin prédite de cette phase de sommeil 3 (N3) ; éventuellement, dans lequel, lorsqu'il est estimé que la personne est en phase de sommeil 3 (N3), le dispositif de traitement est configuré pour arrêter le mouvement de balancement ou de va-et-vient du lit démarré sur la base de la première instruction ou du premier indicateur si la durée de cette phase de sommeil 3 (N3) dépasse les 60 minutes environ ou dépasse une durée prédéterminée.

5. Système selon une quelconque revendication précédente, dans lequel les un ou plusieurs dispositifs améliorant le sommeil sont programmables avec une première valeur d'entrée prédéterminée pour la phase de sommeil 3 (N3), et le mouvement de balancement ou de va-et-vient du lit est réglé sur la première valeur d'entrée prédéterminée vers la fin prédite de la phase de sommeil 3 (N3).

6. Système selon la revendication 5, dans lequel si la première valeur d'entrée prédéterminée est insuffisante pour prolonger une première période de phase de sommeil 3 (N3), le système est configuré pour utiliser une valeur d'entrée supérieure pour le balancement ou le mouvement de va-et-vient du lit pendant une ou plusieurs périodes ultérieures de phase de sommeil 3 (N3).

7. Système selon une quelconque revendication précédente, dans lequel les un ou plusieurs dispositifs améliorant le sommeil sont programmables avec une seconde valeur d'entrée prédéterminée pour la phase de sommeil 4 (REM), et le mouvement de balancement ou de va-et-vient du lit est réglé sur la seconde valeur d'entrée prédéterminée au milieu prédit de la phase de sommeil 4 (REM).

8. Système selon la revendication 7, dans lequel si la seconde valeur d'entrée prédéterminée est insuffisante pour mettre fin prématurément à une première période de phase de sommeil 4 (REM), le système est configuré pour utiliser une valeur d'entrée supérieure pour le mouvement de balancement ou de va-et-vient du lit pendant une ou plusieurs périodes ultérieures de phase de sommeil 4 (REM).

9. Système selon une quelconque revendication précédente, dans lequel les un ou plusieurs dispositifs améliorant le sommeil sont programmables avec l'un et/ou l'autre élément parmi :
des informations cliniques pour la personne (12), et un quelconque ou plusieurs des paramètres d'entrée suivants sont commandés, au moins en partie, en fonction des informations cliniques : type d'entrée, intensité d'entrée, heure de début d'entrée, heure de fin d'entrée, durée d'entrée, motif d'entrée ; et
une valeur de durée de sommeil qui est mesurée à partir d'une heure donnée ou d'un moment où il est détecté que la personne est endormie, et dans lequel une phase de sommeil 3 (N3) ou une phase de sommeil 4 (REM) donnée est prédite comme étant la dernière phase de sommeil 3 (N3) ou la dernière phase de sommeil 4 (REM) avant que la valeur de durée de sommeil ne soit dépassée, les un ou plusieurs dispositifs améliorant le sommeil et l'entrée associée sont arrêtés et ne redémarrent pas lorsque la personne entre dans une phase de sommeil ultérieure avant de se réveiller.

10. Système selon une quelconque revendication précédente, utilisant une configuration cloud, dans lequel des données capturées en temps réel provenant des un ou plusieurs capteurs sont envoyées vers le cloud, éventuellement accompagnées de la vitesse ou d'autres paramètres instantanés de mouvement oscillatoire du lit (10), et des commandes/instructions en temps réel avec des paramètres de mouvement du lit, tels que des informations de vitesse de mouvement oscillatoire du lit, sont envoyées du cloud au lit (10) en mouvement.

11. Support non transitoire lisible par ordinateur contenant des instructions de programme destinées à amener à un dispositif de traitement à exécuter un procédé de commande d'un dispositif ou système améliorant le sommeil qui est un dispositif ou système de balancement de lit pour améliorer la qualité du sommeil, les instructions incluant :
des instructions pour estimer, sur la base des informations provenant d'au moins un ou plusieurs capteurs (12, 14, 16, 18, 20, 22), à quelle phase de sommeil se trouve une personne (12), la phase de sommeil étant sélectionnée dans un groupe comprenant : phase de sommeil 3 (N3), phase de sommeil 4 (REM) et une ou plusieurs autres phases de sommeil ; et
des données de commande de mouvement de lit incluant des instructions ou des indicateurs concernant un, certains ou la totalité des éléments suivants : quand démarrer un mouvement de balancement ou de va-et-vient du lit, quand arrêter un mouvement de balancement ou de va-et-vient du lit et quand ajuster un mouvement de balancement ou de va-et-vient du lit, les données de commande de mouvement de lit incluant l'un et/ou l'autre élément parmi :
une première instruction ou un premier indicateur correspondant à une fin prédite de la phase de sommeil 3 (N3) pour la personne, et
une seconde instruction ou un second indicateur correspondant à un milieu prédit de la phase de sommeil 4 (REM) pour la personne ;
des instructions pour, lorsqu'il est estimé que la personne (12) est en phase de sommeil 3 (N3) et/ou lorsqu'il est estimé que la personne (12) est en phase de sommeil 4 (REM), effectuer l'un et/ou l'autre des éléments suivants en fonction des données de commande de mouvement de lit :
sur la base de la première instruction ou du premier indicateur, démarrer le dispositif ou système améliorant le sommeil vers la fin prédite de la phase de sommeil 3 (N3) afin de commencer à fournir un mouvement de balancement ou de va-et-vient du lit en tant qu'entrée qui agit sur la personne (12) ; et
sur la base de la seconde instruction ou du second indicateur, démarrer le dispositif ou système améliorant le sommeil vers le milieu prédit de la phase de sommeil 4 (REM) pour commencer à fournir un mouvement de balancement ou de va-et-vient du lit en tant qu'entrée qui agit sur la personne (12).

12. Support non transitoire lisible par ordinateur selon la revendication 11, incluant une première valeur d'entrée prédéterminée pour la phase de sommeil 3 (N3), et le mouvement de balancement ou de va-et-vient du lit est réglé sur la première valeur d'entrée prédéterminée vers la fin prédite de la phase de sommeil 3 (N3).

13. Support non transitoire lisible par ordinateur selon la revendication 12, dans lequel, si la première valeur d'entrée prédéterminée est insuffisante pour prolonger une première période de phase de sommeil 3 (N3), une valeur d'entrée supérieure est utilisée pour le mouvement de balancement ou de va-et-vient du lit pendant une ou plusieurs périodes ultérieures de phase de sommeil 3 (N3).

14. Support non transitoire lisible par ordinateur selon l'une quelconque des revendications 11 à 13, incluant une seconde valeur d'entrée prédéterminée pour la phase de sommeil 4 (REM), et le mouvement de balancement ou de va-et-vient du lit est réglé sur la seconde valeur d'entrée prédéterminée au milieu prédit de la phase de sommeil 4 (REM).

15. Support non transitoire lisible par ordinateur selon la revendication 14, dans lequel, si la seconde valeur d'entrée prédéterminée est insuffisante pour mettre fin prématurément à une première période de phase de sommeil 4 (REM), une valeur d'entrée supérieure est utilisée pour le mouvement de balancement ou de va-et-vient du lit pendant une ou plusieurs périodes ultérieures de phase de sommeil 4 (REM).
